# EUROPEAN PATENT SPECIFICATION

(11) **EP 3 503 926 B1**
(45) Date of publication and mention of the grant of the patent: **07.06.2023**
(21) Application number: 17768518.7
(22) Date of filing: 21.08.2017
(51) Int. Cl.: A61K 9/00, A61K 9/06, A61K 9/48, A61K 9/107, A61K 9/08, A61K 47/14, A61K 47/34, A61K 31/216, A61P 1/00, A61P 1/10, A61P 1/12, A61P 1/14

(54) **NEW IMPROVED COMPOSITION OF RACECADOTRIL**
NEUE VERBESSERTE ZUSAMMENSETZUNG VON RACECADOTRIL
NOUVELLE COMPOSITION AMÉLIORÉE DE RACECADOTRIL

(30) Priority: 23.08.2016 SE 1651126
(43) Date of publication of application: 03.07.2019
(73) Proprietor: Johnson & Johnson Consumer Inc., Skillman, NJ 08558 (US)
(72) Inventor: MUHAMMED, Salih Muhsin, 251 09 Helsingborg (SE); LINDELL, Katarina, 251 09 Helsingborg (SE)
(74) Representative: Carpmaels & Ransford LLP
(86) International application number: PCT/IB2017/055041
(87) International publication number: WO 2018/037331

(56) References cited:
- US-A1- 2014 005 262
- US-A1- 2015 342 882
- US-A1- 2016 120 834
- Anonymous: "Handbook of Pharmaceutical Controlled Release Technology", 2000, Marcel Dekker, Inc., XP002774668, page 465

## Description

### FIELD OF INVENTION

The invention relates to an immediate and sustained release composition, a dose unit or a two compartment package comprising the composition as well as the composition, dose unit or two compartment package for use in a method of treating a subject suffering from a disease or disorder in the gastro intestinal tract selected from Irritable Bowel Syndrome, diarrhea, constipation, or bloating, discomfort or pain caused by excessive gas.

### BACKGROUND OF INVENTION

Diarrhea is an intestinal disorder that is characterized by an increase in the frequency of watery bowel movements. It may result from a variety of causes including bacteria or viral induced diarrhea. Food intolerance caused by allergy or the consumption of foods such as fatty or spicy foods may result in diarrhea. Food poisoning may also lead to diarrhea. In some instances, diarrhea may be a symptom of other conditions and diseases. One example is the irritable bowel syndrome (IBS). A patient with IBS typically presents clinically with one of three variants: i) chronic abdominal pain and constipation (also known as spastic colitis); ii) chronic intermittent diarrhea, often without pain; or iii) both features, in an alternating cycle of constipation and diarrhea.

Diarrhea is symptomatic of an intestinal or other bodily function disorder. Various prescription and nonprescription products can be taken for relief. However, many of these products provide relief with some side effects.

Racecadotril is used in the treatment of diarrhea. It reduces (i) hypersecretion of water and electrolytes into the intestinal lumen, (ii) the incidence and duration of acute diarrhea and (iii) diarrhea-associated symptoms.

Simethicone is an orally administered anti-foaming agent used to reduce bloating, discomfort or pain caused by excessive gas, mainly swallowed air, with small amounts of hydrogen and methane in the stomach or intestines.

US2016/0120834 discloses a method to manufacturing cadotril particles that could be formulated either as an immediate or a sustained release formulation, i.e., tablet or liquid formulation as shown in the examples.

US2015/342882 relates to methods of treatment using cadotril compositions.

There is a need for new products containing either racecadotril or an enantiomer of racecadotril or mixtures thereof or a combination with simethicone to be able to provide new products on the market which aim at helping consumers suffering from a disorder or disease within the gastro intestinal tract.

### SUMMARY OF THE INVENTION

The invention relates to the development of new improved compositions comprising racecadotril or an enantiomer of racecadotril or mixtures thereof alone or in combination with Simethicone.

It has surprisingly been found that such a composition will provide an immediate as well as s sustained release profile, which enables the possibility to get a faster relief in a subject suffering from a disease or disorder in the gastro intestinal tract as well as a sustained relief which reduces the number of doses to be taken daily. In addition the use of few and less toxic excipients it is as well possible to obtain a composition that is not harmful for the subject. Further the formulations with oil allow better taste masking of the active ingredient, which enables direct administration into the mouth.

In a first aspect the invention relates to an immediate and sustained release composition or a two compartment package comprising a first immediate release composition comprising at least racecadotril or an enantiomer of racecadotril or mixtures thereof in an amount of at most 10 % w/w and at least one liquid-lipid excipient present in an amount of from at least 90 % w/w of the total amount of the first composition and s second sustained release composition comprising at least racecadotril or an enantiomer of racecadotril or mixtures thereof in an amount of from 10 % to 40 % w/w and at least one liquid-lipid excipient present in an amount of 60 % to 90 % w/w of the total amount of the second composition, wherein the liquid-lipid excipient is at least one medium chain triglycerides or at least an oil containing medium chain triglycerides or mixtures thereof.

In a second aspect the invention relates to a an immediate and sustained release composition or a two compartment package comprising a first immediate release composition comprising at least racecadotril or an enantiomer of racecadotril or mixtures thereof in an amount of at most 10 % w/w of the total amount of the first composition and at least one liquid-lipid excipient present in an amount of from at least 40 % w/w of the total amount of the first composition and simethicone in an amount of at most 50 % w/w of the total amount of the first composition and second sustained release composition comprising at least racecadotril or an enantiomer of racecadotril or mixtures thereof in an amount of from 10 % to 50 % w/w of the total amount of the second composition at least one liquid-lipid excipient present in an amount of from 10 % to 80 % w/w of the total amount of the second composition and simethicone in an amount of 10 % to 60 % w/w of the total amount of the second composition, wherein the liquid-lipid excipient is at least one medium chain triglycerides or at least an oil containing medium chain triglycerides or mixtures thereof.

Such a composition or package will give rise to the possibility to provide higher concentrations/doses of racecadotril or an enantiomer of racecadotril or mixtures thereof compared to what is possible today as well as due to the increased bioavailability decrease the concentration/dose. By providing a prolonged release profile it will secure that drug level will be kept within the effective concentration range for a longer period of time. Such prolonged release profile makes it possible to reduce dosing frequency as well as having once or twice daily dosing instead of three times daily dosing, which most products provide on the market today. In addition, the prolonged release may help to avoid too high plasma concentration, which could lead to increased risk for adverse or unwanted effects. There is no product available on the market today providing such a product and which solves the above identified problems.

Finally, the invention relates to the compositions and packages as defined above and below in the application for use in a method of treatment of a subject suffering from a disease or disorder in the gastro intestinal tract selected from Irritable Bowel Syndrome, diarrhea, constipation, or bloating, discomfort or pain caused by excessive gas, wherein the semi-solid could be co-administrated with one or more dietary fibre products.

### BRIEF DESCRIPTION OF THE DRAWINGS

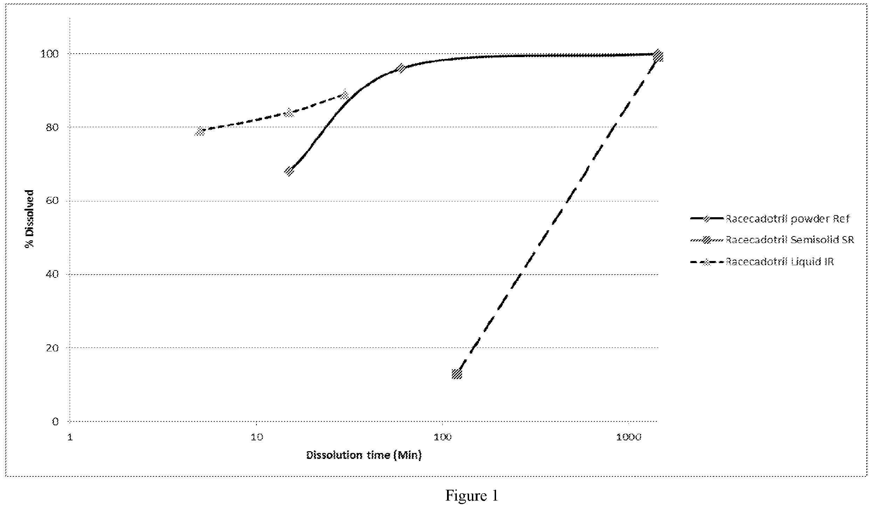

Figure 1: Racecadotril formulations release tests for liquid immediate release (IR), semisolid sustained release (SR) formulations and reference racecadotril formulation (powder or granule filled capsule).

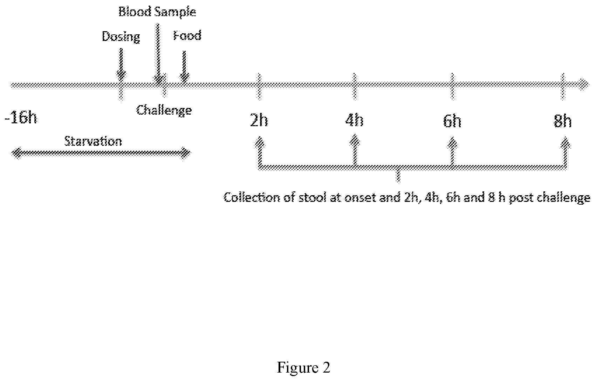

Figure 2: Pharmacodynamic studies in Castor oil diarrhea model in rats; chronological sequence of the study method.

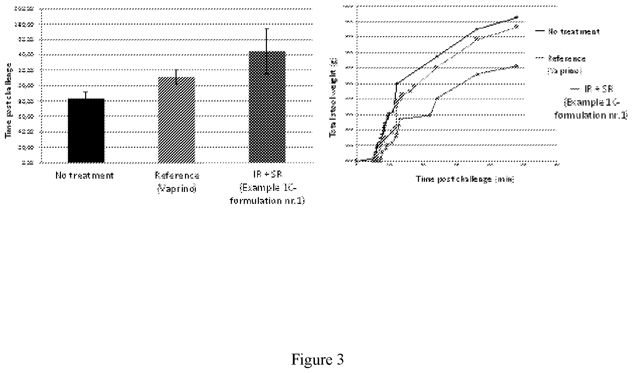

Figure 3: Results of pharmacodynamic studies in Castor oil diarrhea model in rats; study of IR plus SR formulation compared to reference racecadotril formulation (powder or granule filled capsule). Evaluation of Time to onset (Time from challenge to first diarrhea) and Accumulated total stool wet weight (collected after castor oil challenge).

### DETAILED DESCRIPTION OF EMBODIMENTS OF THE INVENTION

### Definitions

In the context of the present application and invention the following definitions apply:
Racecadotril also known as acetorphan, chemically known as benzyl N-[3-(acetylthio)-2 benzylpropanoyl] glycinate is an anti-diarrheal drug which acts as a peripherally acting enkephalinase inhibitor. It has an anti-secretory effect and used to treat diarrhoea. It reduces the secretion of water and electrolytes into the intestine. Racecadotril is a prodrug and it is hydrolysed to its active metabolite, thiorphan following intravenous or oral administration. Racecadotril may be in the R-form or S-form or a mixture thereof. Thiorphan is the active metabolite of racecadotril, which exerts the bulk of its inhibitory actions on enkephalinase.

The term "racemic" is intended to mean an equimolar mixture of enantiomers.

The term "enantiomer" is intended to mean a stereoisomer that is related like an object and its mirror reflection. Enantiomers occur only with compounds whose molecules are chiral, that is, with molecules that are not superposable on their mirror reflections. Separate enantiomers rotate the plane of polarized light and are said to be optically active. They have equal but opposite specific rotation. Examples of enantiomers are ecadotril and dexecadotril.

As used herein, the term "sustained release" ("SR") refers to compositions which are characterized by having at least one of the active components (i.e., racecadotril) having a release over a period of at least about 5 hours. As with formulations described herein, "sustained release" may be achieved by a single formulation containing both "immediate release" components and a "sustained release" (i.e., release for about 5 hours). The release profile may be assessed via *in vitro* dissolution using techniques known to those of skill in the art (e.g., USP basket method, Paddle Method, channel flow method, or other methods known in the literature). The release profile can be assessed *in vivo* (e.g., for bioavailability determinations), using plasma concentrations to assess maximum plasma concentration (Cₘₐₓ) and area under the curve (AUC). Such assays are well known to those of skill in the art.

The term "immediate release" ("IR") is intended to mean the release of an active ingredient (e.g., racecadotril) from a pharmaceutical formulation where the rate of release of the active pharmaceutical ingredient from the pharmaceutical formulation is not retarded by means of a controlled release matrix or other such means and where the components of the pharmaceutical formulation are designed such that, upon ingestion, maximum exposure of said active pharmaceutical ingredient to body tissues occurs in the minimum period of time. As described herein, an "immediate release" component preferably releases in less than 1 hour.

The term Medium Chain Triglyceride(s) (MCTs) is/are intended to mean triglycerides whose fatty acids have an aliphatic tail of 6-12 carbon atoms.

The fatty acids found in MCTs are called medium-chain fatty acids (MCFAs). Like all triglycerides, MCTs are composed of a glycerol backbone and three fatty acids.

The term "substantially free from water or free from water" is intended to mean that the content of water present in the composition is less than about 2 wt.% based on the total wt.% of the composition, such as less than 1.5,1, 0.5, 0.4, 0.3, 0.2 or less than 0.1 or totally free from water, i.e., 0 wt% based on the total wt.% of the composition.

The term "substantially free from non-ionic surfactants or free from non-ionic surfactants" is intended to mean that the content of the non-ionic surfactant present in the composition is less than about 2 wt.% based on the total wt.% of the composition, such as less than 1.5,1, 0.5, 0.4, 0.3, 0.2 or less than 0.1 or totally free from surfactant, i.e., 0 wt.% based on the total wt.% of the composition. The definition of a non-ionic surfactant is well-known for a person skilled in the art as being compounds that lower the surface tension (or interfacial tension) between two liquids or between a liquid and a solid. Non-ionic surfactants are amphiphilic molecules that have both a hydrophobic group non-polar "tail" and a hydrophilic group polar but uncharged "head".

The term "%w/w" is intended to mean the percentage of an ingredient(s)/ the total percentage by weight of the composition (100 %).

The term "bioavailability" is intended to mean, the rate and extent to which the active substance or active moiety is absorbed from a pharmaceutical form and becomes available at the site of action. Bioavailability of oral racecadotril is assessed by monitoring concentrations of racecadotrils active metabolite (thiorphan) in the general circulation.

A "dosage", "dosage form", "dose unit" or "dose" as used herein means the amount of a pharmaceutical formulation comprising therapeutically active agent(s) administered at a time. "Dosage", "dosage form", "dose unit" or "dose" includes administration of one or more units of pharmaceutical formulation administered at the same time.

### The immediate and sustained release composition

The invention relates to a composition comprising an immediate and a sustained release composition
In one embodiment the first immediate release composition comprises at least racecadotril or an enantiomer of racecadotril or mixtures thereof in an amount of at most 10 % w/w, such as 9, 8, 7, 6, 5, 4, 3, 2, 1, 0,5 % w/w of the total amount of the first composition and at least one liquid-lipid excipient present in an amount of from at least 90 % w/w, such as 91, 92, 93, 94, 95 ,96, 97, 98, 99, 99,5 %w/w of the total amount of the first composition, wherein the liquid-lipid excipient is at least one medium chain triglycerides or at least an oil containing medium chain triglycerides or mixtures thereof.

The immediate release composition may be a Self-Emulsifying Nano-Emulsion of racecadotril selected from the formulations disclosed in US20150342882, i.e., contains surfactants. The immediate release composition may for example contain one or more surfactants, such as Polyoxyl 60 hydrogenated castor oil (sold under the trade mark CREMOPHOR RH 60).

The second sustained release composition comprising at least racecadotril or an enantiomer of racecadotril or mixtures thereof in an amount of from 10 % to 40 % w/w, such as about 10 to about 30, about 10 to about 20, about 10 to about 35, about 20 to about 30, about 20 to about 35, such as about 10, 15, 20, 25 , 30, 35 or 4 % w/w of the total amount of the second composition and at least one liquid-lipid excipient present in an amount of 60 % to 90 % w/w, such as about 60 to about 80, about 60 to about 70, about 70 to about 90, about 70 to about 80 % w/w of the total amount of the second composition, wherein the liquid-lipid excipient is at least one medium chain triglycerides or at least an oil containing medium chain triglycerides or mixtures thereof.

In another embodiment the first immediate release composition comprises at least racecadotril or an enantiomer of racecadotril or mixtures thereof in an amount of at most 10 % w/w, such as 9, 8, 7, 6, 5, 4, 3, 2, 1, 1,5 % w/w of the total amount of the first composition and at least one liquid-lipid excipient present in an amount of from at least 90 % w/w, such as 91, 92, 93, 94, 95 ,96, 97, 98, 99, 99,5 %w/w of the total amount of the first composition, wherein the liquid-lipid excipient is at least one medium chain triglycerides or at least an oil containing medium chain triglycerides or mixtures thereof. In addition the first immediate release composition comprises simethicone in an amount of at most 50% w/w, such as 45, 40, 35, 30, 25, 20, 15, 10, 5 % w/w of the total amount of the first composition.

The second sustained release composition comprising at least racecadotril or an enantiomer of racecadotril or mixtures thereof in an amount of from 10 % to 40 % w/w, such as about 10 to about 30, about 10 to about 20, about 10 to about 35, about 20 to about 30, about 20 to about 35, such as about 10, 15, 20, 25 , 30, 35 or 4 % w/w of the total amount of the second composition and at least one liquid-lipid excipient present in an amount of 60 % to 90 % w/w, such as about 60 to about 80, about 60 to about 70, about 70 to about 90, about 70 to about 80 % w/w of the total amount of the second composition, wherein the liquid-lipid excipient is at least one medium chain triglycerides or at least an oil containing medium chain triglycerides or mixtures thereof. In addition the second sustained release composition comprises simethicone in an amount of 10 % to 60 % w/w, such as about 15 to about 50, about 40 to about 50 % w/w of the total amount of the second composition.

Simethicone may be available from DOW CORNING(R) Q7-2243 LVA, SIMETHICONE USP, or DOW CORNING Antifoam M.

The liquid-lipid excipient used in the immediate and sustained release composition in any of the compartment is at least one medium chain triglyceride (MCT) or at least one oil containing medium chain triglycerides or a mixture thereof.

The immediate and sustained release composition may be substantially free from non-ionic surfactants and/or being substantially free from water as defined above. By not utilizing any non-ionic surfactant, there will be no problem with unpleasant taste, irritation or toxic effect will occur which is common upon using surfactants.

The at least one medium chain triglyceride (MCT), i.e., an ester of glycerol and a medium chain fatty acid or a natural oil containing medium chain fatty acids, wherein the medium chain fatty acids are selected from the group consisting of caprylic acid (C8), caproic (C6) acid, capric acid (C10), lauric acid (C12) or mixture thereof. One example being that the medium chain triglyceride is an ester of glycerol and one or more medium chain fatty acids being caprylic or capric acid or a mixture thereof. Other examples are found in table 1.

The Medium chain fatty acid (MCFA), is one or more medium chain fatty acids selected from the group consisting of caprylic acid (C8), caproic (C6) acid, capric acid (C10), lauric acid and esters of caprylic acid (C8), caproic (C6) acid, capric acid (C10), lauric acid. The MCFA may be a mixture of caprylic and capric acid. The amount of the MCFA's in a MCT may be C6 < 2.0 %, C8 about 50-80 %, C10 about 20-50 %. One example is C12 <3.0 % and C14 <1.0% and the total amount of C8 and C10 up to 95% and the water content <0.2%.

Examples of MCTs presented on the market today are shown in table 1 below.

**Table 1. Examples of commercial MCT.**

| Product-Trade Mark | Supplier | Chemical description/INCI name | Listed in |
|---|---|---|---|
| Crodamol GTCC-LQ-(MV) | Croda | Caprylic/Capric Triglyceride | Triglycerides, Medium-Chain PhEur; Medium-Chain Triglycerides NF (Caprylic/Capric Triglycerides |
| Old Trade Mark: Estasan GT8-60 3575 | | | |
| MIGLYOL 812 | Sasol | Caprylic / Capric Triglyceride | Ph. Eur., USP-NF, JPE, DMF |
| LABRAFAC LIPOPHILE WL 1349 | Gattefossé | Triglycerides medium-chain EP/Medium-chain triglycerides NF/Medium chain fatty acid triglyceride JPE | DMF |
| | | | USP/NF |
| | | | EP |
| | | | JP/JPE |
| NEOBEE^{®} M-5 | STEPAN | Captrin, Medium Chain Triglycerides, Caprylic/Capric Triglycerides or Glyceryl Tri(caprylate/caprate). | Complies with the specifications for Medium Chain Triglycerides of the National formulary as published by the U.S. Pharmacopoeia (USP 27/NF 22) and with EP and JPE. NEOBEE M-5 is Kosher and Halal Certified. NEOBEE M-5 has a Type IV Drug Master File (DMF) available. |
| CAPTEX^{®} 355 | Abitec Corporation | Glycerol Tricaprylate/Caprate, Medium Chain Triglyceride (MCT); Caprylic/Capric Triglyceride; Octanoic/Decanoic Acid, Triglyceride | Meets current European Pharmacopoeia for Triglycerides, Medium-Chain, United States Pharmacopeia/National Formulary for Medium-Chain Triglyerides and Japanese Pharmaceutical Excipients monographs for Medium Chain (Fatty Acid) Triglycerides. |

The MCTs shown in table 1 above can be purchased from the following companies. Miglyol 812 from SASOL GmbH, CRODAMOL GTCC from Croda, or Neobees M-5 oil from Stepan and LABRAFAC LIPOPHILE WL 1349 from Gattefossé.

Examples of natural oils that could be used are all natural oils comprising MCTs, such as coconut or palm or palm kernel oils.

The immediate and sustained release compositions may further comprise one or more ingredient(s) selected from the list consisting of coloring agents, antioxidants, flavoring agents, sweeteners, thickeners, emulsifiers, excipients, preservatives and gelling agents.

Additionally, the compositions may comprise one or more fibres, such as dietary fibre which consists of non-starch polysaccharides such as arabinoxylans, cellulose, and many other plant components such as resistant starch, resistant dextrins, inulin, lignin, waxes, chitins, pectins, beta-glucans, and oligosaccharides and other types of carbohydrate that the body can't digest and simply passes through the entire digestive tract. Generally, fibres comes from plant foods: fruits, vegetables, grains, nuts, and legumes such fibre are classified as soluble fibres such as psyllium fibres, others are classified as insoluble fibres like those found in the seeds and skins of fruit as well as whole-wheat bread and brown rice.

The immediate and sustained release composition may further comprise an additional active ingredient. The additional active ingredient may be, a digestive health active ingredient, for example, laxatives, antacids, proton pump inhibitors, anti-gas agents, antiemetics, H2 blockers, a second antidiarrheal agent, antispasmotic agents or analgesic agents and the like. Examples of additional agents includes loperamide, α-galactosidase enzyme, calcium carbonate, aluminum hydroxide and magnesium hydroxide.

The dosage form of the immediate and sustained release composition may be capsules.

### A two compartment package

The invention further relates to a two compartment package comprising racecadotril or an enantiomer of racecadotril or mixtures thereof with or without simethicone.

In a first embodiment the two compartment package comprises a first immediate release composition in a first compartment comprising at least racecadotril or an enantiomer of racecadotril or mixtures thereof in an amount of at most 10 % w/w, such as 9, 8, 7, 6, 5, 4, 3, 2, 1, 1,5 % w/w of the total amount of the first composition and at least one liquid-lipid excipient present in an amount of from at least 90 % w/w, such as 91, 92, 93, 94, 95 ,96, 97, 98, 99, 99,5 %w/w of the total amount of the first composition present in the first compartment and a second composition in a second compartment comprising at least racecadotril or an enantiomer of racecadotril or mixtures thereof in an amount of from 10 % to 40 % w/w, such as about 10 to about 30, about 10 to about 20, about 10 to about 35, about 20 to about 35, such as about 10, 15, 20, 25 , 30, 35 or 4 % w/w of the total amount of the second composition and at least one liquid-lipid excipient present in an amount of 60 % to 90 % w/w, such as about 60 to about 80, about 60 to about 70, about 70 to about 90, about 70 to about 80 % w/w of the total amount of the second composition, wherein the liquid-lipid excipient is at least one medium chain triglycerides or at least an oil containing medium chain triglycerides or mixtures thereof.

In a further embodiment, the invention further relates to a two compartment package comprising racecadotril or an enantiomer of racecadotril or mixtures thereof with simethicone in a first immediate release composition in a first compartment and a second sustained release composition in a second compartment.

The first immediate release composition comprises at least racecadotril or an enantiomer of racecadotril or mixtures thereof in an amount of at most 10 % w/w, such as 9, 8, 7, 6, 5, 4, 3, 2, 1, 1,5 % w/w of the total amount of the first composition and at least one liquid-lipid excipient present in an amount of from at least 90 % w/w, such as 91, 92, 93, 94, 95 ,96, 97, 98, 99, 99,5 %w/w of the total amount of the first composition, wherein the liquid-lipid excipient is at least one medium chain triglycerides or at least an oil containing medium chain triglycerides or mixtures thereof. In addition, the first immediate release composition comprises simethicone in an amount of at most 50% w/w, such as 45, 40, 35, 30, 25, 20, 15, 10, 5 % w/w of the total amount of the first composition.

The second sustained release composition comprising at least racecadotril or an enantiomer of racecadotril or mixtures thereof in an amount of from 10 % to 40 % w/w, such as about 10 to about 30, about 10 to about 20, about 10 to about 35, about 20 to about 35, such as about 10, 15, 20, 25, 30, 35 or 4 % w/w of the total amount of the second composition and at least one liquid-lipid excipient present in an amount of 60 % to 90 % w/w, such as about 60 to about 80, about 60 to about 70, about 70 to about 90, about 70 to about 80 % w/w of the total amount of the second composition, wherein the liquid-lipid excipient is at least one medium chain triglycerides or at least an oil containing medium chain triglycerides or mixtures thereof. In addition, the second sustained release composition comprises simethicone in an amount of 10 % to 60 % w/w, such as about 15 to about 50, about 40 to about 50 % w/w of the total amount of the second composition.

Further components/ingredients present in the package are equal to those disclosed in the composition above.

The two compartment package may be a stick pack, pouch or a sachet.

Racecadotril or an enantiomer of racecadotril or mixtures thereof is/are present in the unit dose or the two compartment package in an amount from about 5 mg to about 200 mg, such as about 5 mg or about 100 mg and simethicone is present in an amount from about 50 to about 1500 mg, such as about 50 to about 1000 mg, such as about 50 to about 500 mg, such as about 50 to about 100 mg, such as about 2 mg to about 150 mg, such as about 6,25 or about 125 mg.

### USE AND METHOD OF TREATMENT

Further the invention relates to the immediate and sustained release composition or the two compartment package defined above for use in the treatment of a subject suffering from a disease or disorder in the gastro intestinal tract, such as Irritable Bowel Syndrome (IBS), such as diarrhea and/or constipation and/or bloating, discomfort or pain caused by excessive gas.

Also disclosed is a method of treatment of a subject suffering from a disease or disorder in the gastro intestinal tract, such as IBS, such as diarrhea and/or constipation and/or bloating, discomfort or pain caused by excessive gas.

### EXAMPLES

### Example 1: Composition and preparation of Immediate release IR, Sustained release SR and Combination (IR plus SR)

### Materials:

Racecadotril powder is available from Sigma-Aldrich, Triglycerides, Medium-Chain PhEur (MCT), Crodamol GTCC-LQ-(MV), was obtained from Croda, Simethicone Q7-2243 LVA, SIMETHICONE USP was obtained from DOW CORNING(R).

### Preparation Method:

Preparation of bulk liquid IR formulation
Racecadotril is weighed in a scintillation vial.

The MCT is weighed and added in the same vial.

The mixture is heated in a water bath at 80°C (the vial is closed to avoid direct contact with water or water vapor) and mixed using stirred using vortex or homogenizer to dissolve racecadotril and achieve a clear homogenous solution.
Cooling down at room temperature
Simethicone can be added either before heating and mixing or to be mixed at the end.

### Preparation of bulk SR semi-solid formulation

Racecadotril is weighed in a scintillation vial.

The MCT (obtained from Croda) is weighed and added in the same vial.

The mixture is heated in a water bath at 80°C (the vial is closed to avoid direct contact with water or water vapor) and mixed using stirred using vortex or homogenizer to dissolve racecadotril and achieve a clear homogenous solution.

Cooling down at room temperature with continuous mixing in order to obtain a semi-solid formulation.

Simethicone can be added either before heating and mixing or to be mixed with the semisolid at the end.

### Preparation of bulk IR Self-Emulsifying Nano-Emulsion

All the ingredients were weighed into a suitable glass bottle with magnetic stir bar and mixed until a clear solution was obtained (^{~}30 hours).

### Example 1A: Immediate release formulations

| Formulation nr. | Composition (% w/w) |
|---|---|
| 1 (IR) | Racecadotril 1%, MCT 99% (IR) |
| 2 (IR) | Racecadotril 1%, Simethicone 1.25%, MCT 97,75% (IR) |
| 3 (IR) | Racecadotril 1%, Simethicone 12.5%, MCT 86.5% (IR) |

### Example 1B: Sustained release formulations

| Formulation nr. | Composition (% w/w) |
|---|---|
| 1 (SR) | Racecadotril 15%, MCT 85% (SR) |
| 2 (SR) | Racecadotril 20%, MCT 80% (SR) |
| 3 (SR) | Racecadotril 25%, MCT 75% (SR) |
| 4 (SR) | Racecadotril 15%, Simethicone 18.75%, MCT 66,25% (SR) |
| 5 (SR) | Racecadotril 20%, Simethicone 25% in MCT 55% (SR) |
| 6 (SR) | Racecadotril 25%, Simethicone 31.25% in MCT 43.75% (SR) |
| 7 (SR) | Racecadotril 10%, Simethicone 20%, MCT 70% (SR) |
| 8 (SR) | Racecadotril 10%, Simethicone 40%, MCT 50% (SR) |
| 9 (SR) | Racecadotril 10%, Simethicone 60%, MCT 30% (SR) |

### Example 1C: Immediate release (IR) plus sustained release (SR) Combination formulations

| Formulation nr. | Composition (%) |
|---|---|
| 1) | Racecadotril 1%, Simethicone 1.25%, MCT 97,75% (IR) |
| 2 (IR)+ 4 (SR) | + |
| | Racecadotril 15%, Simethicone 18.75%, MCT 66,25% (SR) |

### Example 1D: Immediate release (IR) Self-Emulsifying Nano-Emulsion

| Ingredients | (%w/w) | | | | |
|---|---|---|---|---|---|
| Formula | a | b | c | d | e |
| Racecadotril | 9.40 | 8.86 | 8.04 | 7.79 | 7.40 |
| Polyoxyl 35 Castor oil (Super Refined Etocas^{®} 35; NF, EP, JP) | 79.71 | 52.85 | 27.58 | 18.44 | 9.26 |
| Glyceryl Caprylate NF (Mono-, Di-glycerides; Imwitor^{®} 988; NF, EP, JP) | 9.06 | 36.47 | 62.52 | 71.91 | 81.44 |
| Medium Chain Triglycerides (Miglyol^{®} 812N; NF, EP, JP) | 1.83 | 1.82 | 1.86 | 1.86 | 1.90 |
| Total | 100.00 | 100.00 | 100.00 | 100.00 | 100.00 |

Example 2: Racecadotril formulations release tests for liquid immediate release (IR), semisolid sustained release (SR) formulation and reference racecadotril (powder or granule filled capsule formulation).

Dissolution test was accomplished using paddle dissolution tester. Liquid formulation was added by a syringe whereas sinker baskets JP13 were used for capsules of semisolid and powder formulations. Dissolution media was a phosphate buffer pH 6.2 with 1% SDS at 37°C temperature.

Samples of 1.5ml were taken and filtered using Nylon membrane 0.45µm. Racecadotril concentration was determined using Ultra Performance Liquid Chromatography UPLC.

### Example 3: Addition of flavor and sweetener to racecadotril formulations both liquid immediate release (IR) and semisolid sustained release (SR)

| | Formulation (g) | Balsamic Mint (mg) | Sucralos e (mg) |
|---|---|---|---|
| Racecadotril Liquid 1% in MCT IR (Example 1 Formulation nr.1) | 5 | 50 | 200 |
| Racecadotril Semisolid 15% Simethicone 18,75% in MCT SR (Example 1 Formulation nr.4) | 6,68 | 62 | 190 |

Commercially available Balsamic Mint flavor and Sucralose is available from Sigma-Aldrich,

Example 4: Pharmacodynamic studies in Castor oil diarrhea model in rats:
racecadotril Combination formulation (Example 1C) liquid immediate release (IR) plus semisolid sustained release (SR) in comparison with semisolid sustained release (SR) alone, Vaprino^{®}100mg capsule was used as reference.

### The castor oil test - Background

The induction of diarrhea with castor oil results from the action of ricinoleic acid formed by hydrolysis of the oil (Iwao and Terada, 1962, J. Pharmacol 12:137-145). Ricinoleic acid produces changes in the transport of water and electrolytes resulting in a hypersecretory response (Ammon et al., 1974, J. Clin. Invest. 53:374-379). In addition to hypersecretion, ricinoleic acid sensitizes the intramural neurons of the gut. The castor oil test (Niemegeers et al., 1984, Drug Dev. Res. 1:1-20) has widely been used to study effects of antidiarrheals in various preclinical settings. Racecadotril and thiorphan have previously been shown to inhibit castor-oil induced diarrhea in rats (Marcais-Collado et al., 1987, Eur. J. Pharmacol. 144:125-132). Intravenous administration of thiorphan resulted in a dose-dependent protection against diarrhea as measured by cumulative stool weight and time to onset. Protection was most prominent during the first two hours following challenge with castor oil. Similarly, pre-treatment with racecadotril (p.o.), resulted in a reduction in cumulative stool weight following challenge with castor oil. Time to onset was also significantly delayed, although a dose-response relationship could not be established.

### Inhibition of castor-oil induced diarrhea in rats

### Overall study design

The castor oil test described by Niemeegers et al. (1984) was used with small modifications. Following the various treatments, overnight fasted rats received a standard dose of castor oil administered orally by gavage and were caged individually. The time to onset of diarrhea and cumulative stool weights were noted during an 8 h observation period.

### Animals

Male Sprague Dawley rats were obtained from Janvier Labs and housed as described in section 9.2.2. The rats were acclimatized to the housing conditions for at least 7 days before the start of experiments. The approximate weight of animals was 300 g at performance of experiments.

### Experimental procedures in vivo

### Castor oil induced diarrhea

Food (but not water) was withheld 16 h prior to dosing of castor oil (challenge). For induction of diarrhea, the rats received 2 ml of castor oil, delivered by oral gavage. After the challenge, the rats were placed in individual cages with grilled floor. The rats had access to food from 30 minutes after dosing with castor oil and during the whole observation period. Stools were collected on non-wetting paper placed beneath the grilled floor at predefined time points, weighed and thereafter incubated at 70 C for at least 16 hours for determination of dry weight.

The following parameters were evaluated:
Time to onset (first diarrhea)
Accumulated weight of total stool (wet weight and dry weight)
Treatment

The rats were dosed p.o. with racecadotril/vehicle one hour before challenge with castor oil. The rats were slightly sedated using isofluorane at delivery of the test items. The racecadotril dose of 20mg/kg body weight was given based on an assumed rat weight 300g. Animals were weighed before the experiments to allow calculation of the actual delivered dose. 2 ml of saline was delivered by gavage immediately after dosing. Control groups consisting of rats given castor oil only and non-diarrheic rats (given saline instead of castor oil) were included in all experiments. The studies were generally performed over three experimental days and all groups contained rats from all three experimental days to avoid interference from potential day to day variation.

### Method:

Fasting overnight
Administration of racecadotril
Challenge with castor oil 1 h post dosing
Assessment of
   - time to diarrhea onset
   - accumulated stool weight
Blood sampling 1 h post dosing to verify uptake

### Results:

Significant improved effect for IR + SR (Example 1C-formulation nr. 1) compared to reference (Vaprino^{®}100mg capsule). SR alone was not significant compared to reference

## Claims

1. An immediate and sustained release composition comprising
a. a first immediate release composition comprising
i. at least racecadotril or an enantiomer of racecadotril or mixtures thereof in an amount of at most 10 % w/w and
ii. at least one liquid-lipid excipient present in an amount of from at least 90 % w/w of the total amount of the first composition and
b. a second sustained release composition comprising
i. at least racecadotril or an enantiomer of racecadotril or mixtures thereof in an amount of from 10 % to 40 % w/w and
ii. at least one liquid-lipid excipient present in an amount of 60 % to 90 % w/w of the total amount of the second composition
wherein the liquid-lipid excipient is at least one medium chain triglycerides or at least an oil containing medium chain triglycerides or mixtures thereof.

2. The composition according to claim 1, wherein
(a) the racecadotril or an enantiomer of racecadotril or mixtures thereof is/are present in the first composition in an amount of at most 9, 8, 7, 6, 5, 4, 3, 2 or 1 % w/w of the total amount of the first composition and in the second composition in an amount of from 20 % w/w to 30 % w/w of the total amount of the second composition; and/or
(b) at least one liquid-lipid excipient present in an amount of from at least 91, 92, 93, 94, 95 ,96, 97, 98, 99, 99,5 %w/w of the total amount of the first composition and at least one liquid-lipid excipient present in an amount of 60 to 80, 60 to 70, 70 to 90, 70 to 80 % w/w of the total amount of the second composition.

3. An immediate and sustained release composition comprising
c. a first immediate release composition comprising
i. at least racecadotril or an enantiomer of racecadotril or mixtures thereof in an amount of at most 10 % w/w of the total amount of the first composition and
ii. at least one liquid-lipid excipient present in an amount of from at least 40 % w/w of the total amount of the first composition and
iii. simethicone in an amount of at most 50 % w/w of the total amount of the first composition and
d. a second sustained release composition comprising
i. at least racecadotril or an enantiomer of racecadotril or mixtures thereof in an amount of from 10 % to 50 % w/w of the total amount of the second composition
ii. at least one liquid-lipid excipient present in an amount of from 10 % to 80 % w/w of the total amount of the second composition and
iii. simethicone in an amount of 10 % to 60 % w/w of the total amount of the second composition
wherein the liquid-lipid excipient is at least one medium chain triglycerides or at least an oil containing medium chain triglycerides or mixtures thereof.

4. A two compartment package comprising
e. a first immediate release composition in a first compartment comprising
i. at least racecadotril or an enantiomer of racecadotril or mixtures thereof in an amount of at most 10 % w/w and
ii. at least one liquid-lipid excipient present in an amount of from at least 90 % w/w of the total amount of the first composition and
f. a second sustained released composition in a second compartment comprising
i. at least racecadotril or an enantiomer of racecadotril or mixtures thereof in an amount of from 10 % to 40 % w/w and
ii. at least one liquid-lipid excipient present in an amount of 60 % to 90 % w/w of the total amount of the second composition
wherein the liquid-lipid excipient is at least one medium chain triglycerides or at least an oil containing medium chain triglycerides or mixtures thereof.

5. A two compartment package comprising,
g. a first immediate release composition comprising
i. at least racecadotril or an enantiomer of racecadotril or mixtures thereof in an amount of at most 10 % w/w of the total amount of the first composition and
ii. at least one liquid-lipid excipient present in an amount of from at least 40 % w/w of the total amount of the first composition and
iii. simethicone in an amount of at most 50 % w/w of the total amount of the first composition and
h. a second sustained release composition comprising
i. at least racecadotril or an enantiomer of racecadotril or mixtures thereof in an amount of from 10 % to 50 % w/w of the total amount of the second composition
ii. at least one liquid-lipid excipient present in an amount of from 10 % to 80 % w/w of the total amount of the second composition and
iii. simethicone in an amount of 10 % to 60 % w/w of the total amount of the second composition
wherein the liquid-lipid excipient is at least one medium chain triglycerides or at least an oil containing medium chain triglycerides or mixtures thereof.

6. The composition according to any of claims 1-3 or the two compartment package according to claims 4-5, wherein the composition is substantially free from non-ionic surfactants and/or being substantially free from water, wherein the term "substantially free" means that the content of water or the non-ionic surfactant present in the composition is less than 2 wt.% based on the total wt.% of the composition.

7. The composition according to any of claims 1-3 or 6, or the two compartment package according to claims 4-6, wherein the medium chain triglyceride is at least an ester of glycerol and at least one or more medium chain fatty acids selected from the group consisting of caprylic acid (C8), caproic (C6) acid, capric acid (C10), lauric acid (C12) or mixture thereof, preferably wherein the medium chain fatty acids are caprylic or capric acid or a mixture thereof.

8. The composition according to any of claims 1-3 or 6-7, wherein a) the racecadotril is a racemic or the R-form or the S-form or mixtures thereof, optionally, wherein a) is racecadotril.

9. The two compartment package according to claims 4-7, wherein the racecadotril is a racemic and the enantiomers are ecadotril or dexecadotril or mixtures thereof, optionally wherein a) is racecadotril.

10. The composition according to any of claims 1-2 or 6-8, or the two compartment package according to claims 4, 6-7 or 9, wherein the composition further comprises simethicone.

11. The composition according to any of claims 1-3, 6-8 or 10, or the two compartment package according to claims 4-7 or 9-10, wherein the first and the second compositions in the composition or the first and the second compartments in the two compartment package further comprises one or more ingredient(s) selected from the list consisting of colorings, flavors, sweeteners, thickeners, emulsifiers, antioxidants, preservatives, gelling agents and disintegrants.

12. The composition according to any of claims 1-3, 6-8 or 10-11, or the two compartment package according to claims 4-7 or 9-11, wherein the first and the second compositions in the composition or the first and the second compartments in the two compartment package comprises at least one dietary fibre.

13. A dosage form comprising the composition according to any of claims 1-3, 6-8 or 10-12, wherein the dosage form is capsule.

14. The two compartment package according to claims 4-7 or 9-12, wherein said package is a pouch, stick pack or sachet.

15. The composition according to any of claims 1-3, 6-8 or 10-12, the dosage form according to claim 13, or the two compartment package according to any of claims 4-7, 9-12 or 14, wherein racecadotril or an enantiomer of racecadotril or mixtures thereof is/are present in an amount from 5 mg to 200 mg.

16. The composition according to any of claims 1-3, 6-8 or 10-12, the dosage form according to claim 13, or the two compartment package according to any of claims 4-7, 9-12 or 14, wherein simethicone is present in an amount from 50 mg to 1500 mg.

17. The composition according to any of claims 1-3, 6-8 or 10-12, the dosage form according to claim 13, or the two compartment package according to any of claims 4-7, 9-12 or 14, for use in the treatment of a subject suffering from a disease or disorder in the gastro intestinal tract selected from Irritable Bowel Syndrome, diarrhea, constipation, or bloating, discomfort or pain caused by excessive gas.

## Patentansprüche

1. Zusammensetzung mit sofortiger und anhaltender Freisetzung, umfassend
a. eine erste Zusammensetzung mit sofortiger Freisetzung, umfassend
i. mindestens Racecadotril oder ein Enantiomer von Racecadotril oder Gemische davon in einer Menge von höchstens 10 Gew.-% und
ii. mindestens einen flüssigen Lipidhilfsstoff, der in einer Menge von mindestens 90 Gew.-% der Gesamtmenge der ersten Zusammensetzung vorliegt, und
b. eine zweite Zusammensetzung mit anhaltender Freisetzung, umfassend
i. mindestens Racecadotril oder ein Enantiomer von Racecadotril oder Gemische davon in einer Menge von 10 bis 40 Gew.-% und
ii. mindestens einen flüssigen Lipidhilfsstoff, der in einer Menge von 60 bis 90 Gew.-% der Gesamtmenge der zweiten Zusammensetzung vorliegt,
wobei es sich bei dem flüssigen Lipidhilfsstoff um mindestens ein mittelkettiges Triglycerid oder mindestens ein mittelkettige Triglyceride enthaltendes Öl oder Mischungen davon handelt.

2. Zusammensetzung nach Anspruch 1, wobei
(a) das Racecadotril oder ein Enantiomer von Racecadotril oder Mischungen davon in der ersten Zusammensetzung in einer Menge von höchstens 9, 8, 7, 6, 5, 4, 3, 2 oder 1 Gew.-% der Gesamtmenge der ersten Zusammensetzung und in der zweiten Zusammensetzung in einer Menge von 20 Gew.-% bis 30 Gew.-% der Gesamtmenge der zweiten Zusammensetzung vorliegt/vorliegen und/oder
(b) mindestens ein flüssiger Lipidhilfsstoff in einer Menge von mindestens 91, 92, 93, 94, 95, 96, 97, 98, 99, 99,5 Gew.-% der Gesamtmenge der ersten Zusammensetzung vorliegt und mindestens ein flüssiger Lipidhilfsstoff in einer Menge von 60 bis 80, 60 bis 70, 70 bis 90 oder 70 bis 80 Gew.-% der Gesamtmenge der zweiten Zusammensetzung vorliegt.

3. Zusammensetzung mit sofortiger und anhaltender Freisetzung, umfassend
c. eine erste Zusammensetzung mit sofortiger Freisetzung, umfassend
i. mindestens Racecadotril oder ein Enantiomer von Racecadotril oder Gemische davon in einer Menge von höchstens 10 Gew.-% der Gesamtmenge der ersten Zusammensetzung und
ii. mindestens einen flüssigen Lipidhilfsstoff, der in einer Menge von mindestens 40 Gew.-% der Gesamtmenge der ersten Zusammensetzung vorliegt, und
iii. Simethicon in einer Menge von höchstens 50 Gew.-% der Gesamtmenge der ersten Zusammensetzung, und
d. eine zweite Zusammensetzung mit anhaltender Freisetzung, umfassend
i. mindestens Racecadotril oder ein Enantiomer von Racecadotril oder Gemische davon in einer Menge von 10 bis 50 Gew.-% der Gesamtmenge der zweiten Zusammensetzung
ii. mindestens einen flüssigen Lipidhilfsstoff, der in einer Menge von 10 bis 80 Gew.-% der Gesamtmenge der zweiten Zusammensetzung vorliegt, und
iii. Simethicon in einer Menge von 10 bis 60 Gew.-% der Gesamtmenge der zweiten Zusammensetzung,
wobei es sich bei dem flüssigen Lipidhilfsstoff um mindestens ein mittelkettiges Triglycerid oder mindestens ein mittelkettige Triglyceride enthaltendes Öl oder Mischungen davon handelt.

4. Packung mit zwei Kompartimenten, umfassend
e. eine erste Zusammensetzung mit sofortiger Freisetzung in einem ersten Kompartiment, umfassend
i. mindestens Racecadotril oder ein Enantiomer von Racecadotril oder Gemische davon in einer Menge von höchstens 10 Gew.-% und
ii. mindestens einen flüssigen Lipidhilfsstoff, der in einer Menge von mindestens 90 Gew.-% der Gesamtmenge der ersten Zusammensetzung vorliegt, und
f. eine zweite Zusammensetzung mit anhaltender Freisetzung in einem zweiten Kompartiment, umfassend
i. mindestens Racecadotril oder ein Enantiomer von Racecadotril oder Gemische davon in einer Menge von 10 bis 40 Gew.-% und
ii. mindestens einen flüssigen Lipidhilfsstoff, der in einer Menge von 60 bis 90 Gew.-% der Gesamtmenge der zweiten Zusammensetzung vorliegt,
wobei es sich bei dem flüssigen Lipidhilfsstoff um mindestens ein mittelkettiges Triglycerid oder mindestens ein mittelkettige Triglyceride enthaltendes Öl oder Mischungen davon handelt.

5. Packung mit zwei Kompartimenten, umfassend
g. eine erste Zusammensetzung mit sofortiger Freisetzung, umfassend
i. mindestens Racecadotril oder ein Enantiomer von Racecadotril oder Gemische davon in einer Menge von höchstens 10 Gew.-% der Gesamtmenge der ersten Zusammensetzung und
ii. mindestens einen flüssigen Lipidhilfsstoff, der in einer Menge von mindestens 40 Gew.-% der Gesamtmenge der ersten Zusammensetzung vorliegt, und
iii. Simethicon in einer Menge von höchstens 50 Gew.-% der Gesamtmenge der ersten Zusammensetzung,
h. eine zweite Zusammensetzung mit anhaltender Freisetzung, umfassend
i. mindestens Racecadotril oder ein Enantiomer von Racecadotril oder Gemische davon in einer Menge von 10 bis 50 Gew.-% der Gesamtmenge der zweiten Zusammensetzung
ii. mindestens einen flüssigen Lipidhilfsstoff, der in einer Menge von 10 bis 80 Gew.-% der Gesamtmenge der zweiten Zusammensetzung vorliegt, und
iii. Simethicon in einer Menge von 10 bis 60 Gew.-% der Gesamtmenge der zweiten Zusammensetzung,
wobei es sich bei dem flüssigen Lipidhilfsstoff um mindestens ein mittelkettiges Triglycerid oder mindestens ein mittelkettige Triglyceride enthaltendes Öl oder Mischungen davon handelt.

6. Zusammensetzung nach einem der Ansprüche 1-3 oder Packung mit zwei Kompartimenten nach den Ansprüchen 4-5, wobei die Zusammensetzung weitgehend frei von nichtionischen Tensiden ist und/oder weitgehend frei von Wasser ist, wobei der Begriff "weitgehend frei" bedeutet, dass der Gehalt an Wasser bzw. dem nichtionischen Tensid in der Zusammensetzung weniger als 2 Gew.-%, bezogen auf die gesamten Gew.-%-Gehalte der Zusammensetzung, beträgt.

7. Zusammensetzung nach einem der Ansprüche 1-3 oder 6 oder Packung mit zwei Kompartimenten nach den Ansprüchen 4-6, wobei es sich bei dem mittelkettigen Triglycerid um mindestens einen Ester von Glycerin und mindestens eine oder mehrere mittelkettige Fettsäuren, die aus der Gruppe bestehend aus Caprylsäure (C8), Capronsäure (C6), Caprinsäure (C10), Laurinsäure (C12) oder einer Mischung davon ausgewählt sind, handelt, vorzugsweise wobei es sich bei den mittelkettigen Fettsäuren um Caprylsäure oder Caprinsäure oder eine Mischung davon handelt.

8. Zusammensetzung nach einem der Ansprüche 1-3 oder 6-7, wobei a) das Racecadotril in racemischer Form oder der R-Form oder der S-Form oder Gemischen davon vorliegt, gegebenenfalls wobei a) Racecadotril ist.

9. Packung mit zwei Kompartimenten nach den Ansprüchen 4-7, wobei das Racecadotril racemisch ist und es sich bei den Enantiomeren um Ecadotril oder Dexecadotril oder Gemische davon handelt, gegebenenfalls wobei a) Racecadotril ist.

10. Zusammensetzung nach einem der Ansprüche 1-2 oder 6-8 oder Packung mit zwei Kompartimenten nach den Ansprüchen 4, 6-7 oder 9, wobei die Zusammensetzung ferner Simethicon umfasst.

11. Zusammensetzung nach einem der Ansprüche 1-3, 6-8 oder 10 oder Packung mit zwei Kompartimenten nach den Ansprüchen 4-7 oder 9-10, wobei die erste Zusammensetzung und die zweite Zusammensetzung in der Zusammensetzung bzw. das erste Kompartiment und das zweite Kompartiment in der Packung mit zwei Kompartimenten ferner einen oder mehrere Bestandteile umfassen, die aus der Liste bestehend aus Farbmitteln, Geschmacksstoffen, Süßungsmitteln, Verdickern, Emulgatoren, Antioxidantien, Konservierungsstoffen, Gelierungsmitteln und Sprengmitteln ausgewählt sind.

12. Zusammensetzung nach einem der Ansprüche 1-3, 6-8 oder 10-11 oder Packung mit zwei Kompartimenten nach den Ansprüchen 4-7 oder 9-11, wobei die erste Zusammensetzung und die zweite Zusammensetzung in der Zusammensetzung bzw. das erste Kompartiment und das zweite Kompartiment in der Packung mit zwei Kompartimenten mindestens einen Ballaststoff umfassen.

13. Dosierungsform, umfassend die Zusammensetzung nach einem der Ansprüche 1-3, 6-8 oder 10-12, wobei es sich bei der Dosierungsform um eine Kapsel handelt.

14. Packung mit zwei Kompartimenten nach den Ansprüchen 4-7 oder 9-12, wobei es sich bei der Packung um einen Beutel, ein Stick-Pack oder ein Sachet handelt.

15. Zusammensetzung nach einem der Ansprüche 1-3, 6-8 oder 10-12, Dosierungsform nach Anspruch 13 oder Packung mit zwei Kompartimenten nach einem der Ansprüche 4-7, 9-12 oder 14, wobei Racecadotril oder ein Enantiomer von Racecadotril oder Gemische davon in einer Menge von 5 mg bis 200 mg vorliegt/vorliegen.

16. Zusammensetzung nach einem der Ansprüche 1-3, 6-8 oder 10-12, Dosierungsform nach Anspruch 13 oder Packung mit zwei Kompartimenten nach einem der Ansprüche 4-7, 9-12 oder 14, wobei Simethicon in einer Menge von 50 mg bis 1500 mg vorliegt.

17. Zusammensetzung nach einem der Ansprüche 1-3, 6-8 oder 10-12, Dosierungsform nach Anspruch 13 oder Packung mit zwei Kompartimenten nach einem der Ansprüche 4-7, 9-12 oder 14 zur Verwendung bei der Behandlung eines Individuums, das an einer Erkrankung oder Störung im Magen-Darm-Trakt leidet, die aus Reizdarmsyndrom, Diarrhoe, Verstopfung oder Blähungen, Unbehagen oder Schmerzen infolge von zu viel Gas ausgewählt ist.

## Revendications

1. Composition à libération immédiate et prolongée comprenant
a. une première composition à libération immédiate comprenant
i. au moins du racécadotril ou un énantiomère de racécadotril ou des mélanges correspondants en une quantité d'au plus 10 % p/p et
ii. au moins un excipient liquide-lipide présent en une quantité allant d'au moins 90 % p/p de la quantité totale de la première composition et
b. une deuxième composition à libération prolongée comprenant
i. au moins du racécadotril ou un énantiomère de racécadotril ou des mélanges correspondants en une quantité allant de 10 % à 40 % p/p et
ii. au moins un excipient liquide-lipide présent en une quantité de 60 % à 90 % p/p de la quantité totale de la deuxième composition
l'excipient liquide-lipide étant au moins un triglycéride à chaîne moyenne ou au moins une huile contenant des triglycérides à chaîne moyenne ou des mélanges correspondants.

2. Composition selon la revendication 1,
(a) le racécadotril ou un énantiomère de racécadotril ou des mélanges correspondants étant présents dans la première composition en une quantité d'au plus 9, 8, 7, 6, 5, 4, 3, 2, ou 1 % p/p de la quantité totale de la première composition et dans la deuxième composition en une quantité allant de 20 % p/p à 30 % p/p de la quantité totale de la deuxième composition ; et/ou
(b) au moins un excipient liquide-lipide en une quantité allant d'au moins 91, 92, 93, 94, 95, 96, 97, 98, 99, 99,5 % p/p de la quantité totale de la première composition et au moins un excipient liquide-lipide présent en une quantité de 60 à 80, 60 à 70, 70 à 90, 70 à 80 % p/p de la quantité totale de la deuxième composition.

3. Composition à libération immédiate et prolongée comprenant
c. une première composition à libération immédiate comprenant
i. au moins du racécadotril ou un énantiomère de racécadotril ou des mélanges correspondants en une quantité d'au plus 10 % p/p de la quantité totale de la première composition et
ii. au moins un excipient liquide-lipide présent en une quantité allant d'au moins 40 % p/p de la quantité totale de la première composition et
iii. une siméthicone en une quantité d'au plus 50 % p/p de la quantité totale de la première composition et
d. une deuxième composition à libération prolongée comprenant
i. au moins du racécadotril ou un énantiomère de racécadotril ou des mélanges correspondants en une quantité allant de 10 % à 50 % p/p de la quantité totale de la deuxième composition et
ii. au moins un excipient liquide-lipide présent en une quantité allant de 10 % à 80 % p/p de la quantité totale de la deuxième composition et
iii. une siméthicone en une quantité de 10 % à 60 % p/p de la quantité totale de la deuxième composition
l'excipient liquide-lipide étant au moins un triglycéride à chaîne moyenne ou au moins une huile contenant des triglycérides à chaîne moyenne ou des mélanges correspondants.

4. Paquet à deux compartiments comprenant
e. une première composition à libération immédiate dans un premier compartiment comprenant
i. au moins du racécadotril ou un énantiomère de racécadotril ou des mélanges correspondants en une quantité d'au plus 10 % p/p et
ii. au moins un excipient liquide-lipide présent en une quantité allant d'au moins 90 % p/p de la quantité totale de la première composition et
f. une deuxième composition à libération prolongée dans un deuxième compartiment comprenant
i. au moins du racécadotril ou un énantiomère de racécadotril ou des mélanges correspondants en une quantité allant de 10 % à 40 % p/p et
ii. au moins un excipient liquide-lipide présent en une quantité de 60 % à 90 % p/p de la quantité totale de la deuxième composition
l'excipient liquide-lipide étant au moins un triglycéride à chaîne moyenne ou au moins une huile contenant des triglycérides à chaîne moyenne ou des mélanges correspondants.

5. Paquet à deux compartiments comprenant,
g. une première composition à libération immédiate comprenant
i. au moins du racécadotril ou un énantiomère de racécadotril ou des mélanges correspondants en une quantité d'au plus 10 % p/p de la quantité totale de la première composition et
ii. au moins un excipient liquide-lipide présent en une quantité allant d'au moins 40 % p/p de la quantité totale de la première composition et
iii. une siméthicone en une quantité d'au plus 50 % p/p de la quantité totale de la première composition et
h. une deuxième composition à libération prolongée comprenant
i. au moins du racécadotril ou un énantiomère de racécadotril ou des mélanges correspondants en une quantité allant de 10 % à 50 % p/p de la quantité totale de la deuxième composition
ii. au moins un excipient liquide-lipide présent en une quantité allant de 10 % à 80 % p/p de la quantité totale de la deuxième composition et
iii. une siméthicone en une quantité de 10 % à 60 % p/p de la quantité totale de la deuxième composition
l'excipient liquide-lipide étant au moins un triglycéride à chaîne moyenne ou au moins une huile contenant des triglycérides à chaîne moyenne ou des mélanges correspondants.

6. Composition selon l'une quelconque des revendications 1 à 3 ou paquet à deux compartiments selon les revendications 4 et 5, la composition étant sensiblement exempte de tensioactifs non ioniques et/ou étant sensiblement exempte d'eau, les termes « sensiblement exempte » signifiant que la teneur en eau du tensioactif non ionique présent dans la composition est inférieure à 2 % en poids sur la base du % en poids total de la composition.

7. Composition selon l'une quelconque des revendications 1 à 3 ou 6 ou paquet à deux compartiments selon les revendications 4 à 6, le triglycéride à chaîne moyenne étant au moins un ester de glycérol et au moins un ou plusieurs acides gras à chaîne moyenne choisis dans le groupe constitué par l'acide caprylique (C8), l'acide caproïque (C6), l'acide caprique (C10), l'acide laurique (C12) ou un mélange correspondant, préférablement, les acides gras à chaîne moyenne étant l'acide caprylique ou caprique ou un mélange correspondant.

8. Composition selon l'une quelconque des revendications 1 à 3 ou 6 et 7, a) le racécadotril étant un racémique ou la forme R ou la forme S ou des mélanges correspondants, éventuellement, a) étant le racécadotril.

9. Paquet à deux compartiments selon les revendications 4 à 7, le racécadotril étant un racémique et les énantiomères étant l'écadotril ou le déxécadotril ou des mélanges correspondants, éventuellement, a) étant le racécadotril.

10. Composition selon l'une quelconque des revendications 1 et 2 ou de 6 à 8, ou paquet à deux compartiments selon les revendications 4, 6 à 7 ou 9, la composition comprenant en outre une siméthicone.

11. Composition selon l'une quelconque des revendications 1 à 3, 6 à 8 ou 10, ou paquet à deux compartiments selon les revendications 4 à 7 ou 9 et 10, la première et la deuxième compositions dans la composition ou le premier et le deuxième compartiments dans le paquet à deux compartiments comprenant en outre un ou plusieurs ingrédients choisis dans la liste constituée par des matières colorantes, des arômes, des édulcorants, des épaississants, des émulsifiants, des antioxydants, des conservateurs, des agents gélifiants et des désintégrants.

12. Composition selon l'une quelconque des revendications 1 à 3, 6 à 8 ou 10 et 11, ou paquet à deux compartiments selon les revendications 4 à 7 ou 9 à 11, la première et la deuxième compositions dans la composition ou le premier et le deuxième compartiments dans le paquet à deux compartiments comprenant au moins une fibre alimentaire.

13. Forme de dosage comprenant la composition selon l'une quelconque des revendications 1 à 3, 6 à 8 ou 10 à 12, la forme de dosage étant une capsule.

14. Paquet à deux compartiments selon les revendications 4 à 7 ou 9 à 12, ledit paquet étant une poche, un stick pack ou un sachet.

15. Composition selon l'une quelconque des revendications 1 à 3, 6 à 8 ou 10 à 12, forme de dosage selon la revendication 13, ou paquet à deux compartiments selon l'une quelconque des revendications 4 à 7 ou 9 à 12 ou 14, le racécadotril ou un énantiomère de racécadotril ou des mélanges correspondants étant présents en une quantité de 5 mg à 200 mg.

16. Composition selon l'une quelconque des revendications 1 à 3, 6 à 8 ou 10 à 12, forme de dosage selon la revendication 13, ou paquet à deux compartiments selon l'une quelconque des revendications 4 à 7 ou 9 à 12 ou 14, la siméthicone étant présente en une quantité de 50 mg à 1 500 mg.

17. Composition selon l'une quelconque des revendications 1 à 3, 6 à 8 ou 10 à 12, forme de dosage selon la revendication 13, ou paquet à deux compartiments selon l'une quelconque des revendications 4 à 7 ou 9 à 12 ou 14, pour une utilisation dans le traitement d'un sujet souffrant d'une maladie ou d'un trouble dans le tract gastrointestinal choisi parmi le syndrome de l'intestin irritable, la diarrhée, la constipation ou les ballonnements, un inconfort ou une maladie causé(e) par un excès de gaz.
